# EUROPÄISCHE PATENTSCHRIFT

(11) **EP 3 494 912 B1**
(45) Veröffentlichungstag und Bekanntmachung des Hinweises auf die Patenterteilung: **02.06.2021**
(21) Anmeldenummer: 18210800.1
(22) Anmeldetag: 06.12.2018
(51) Int. Cl.: A61B 17/92, A61B 17/88, B25B 27/18, B25B 15/00, B25B 19/00

(54) **VORRICHTUNG ZUM LÖSEN VON, INSBESONDERE KALTVERSCHWEISSTEN, SCHRAUBEN IN DER ORTHOPÄDIE**
APPARATUS FOR LOOSENING IN PARTICULAR COLD WELDED, SCREWS USED FOR ORTHOPAEDICS
DISPOSITIF DE DESSERRAGE DE VIS, EN PARTICULIER SOUDÉES À FROID, EN ORTHOPÉDIE

(30) Priorität: 06.12.2017 DE 102017128988
(43) Veröffentlichungstag der Anmeldung: 12.06.2019
(73) Patentinhaber: Endo-Passion GmbH, 78573 Wurmlingen (DE); Walter-Konstruktion, 78532 Tuttlingen (DE)
(72) Erfinder: Voigtländer, Thomas, 78573 Wurmlingen (DE); Walter, Christian, 78532 Tuttlingen (DE)
(74) Vertreter: Kuhnen & Wacker Patent- und Rechtsanwaltsbüro PartG mbB

(56) Entgegenhaltungen:
- EP-A1- 2 732 776
- DE-A1-102010 029 559
- GB-A- 2 510 406
- US-A1- 2011 098 715
- US-A1- 2011 270 323

## Beschreibung

### Gebiet der Erfindung

Die Erfindung betrifft eine Vorrichtung zum Lösen von, insbesondere kaltverschweißten, Schrauben in der Orthopädie.

Jährlich werden in deutschen Krankenhäusern über 200.000 künstliche Hüftgelenke und etwa 150.000 künstliche Kniegelenke implantiert (Stand 2011). Die Tendenz der Anzahl dieser Eingriffe ist steigend. Bei künstlichen Gelenken wie Hüftprothesen oder künstlichen Kniegelenken kann dabei mit einer Verweildauer von etwa 15 bis 20 Jahren im Körper gerechnet werden, bevor die künstlichen Gelenke erneuert werden müssen. Aufgrund von Faktoren wie einer steigenden Lebenserwartung sowie der steigenden Anzahl von implantierten künstlichen Gelenken, nimmt auch die Anzahl notwendiger Erneuerungen der Implantate und somit notwendiger Revisionen, das heißt dem Entfernen des zuvor eingesetzten Implantats, zu.

Die auf das Knochenmaterial wirkende Belastung ist durch die in den Knochen eingebrachten Schrauben hoch und steigt beispielsweise mit der Anzahl an nacheinander implantierten künstlichen Gelenken an, da die Verankerung im Knochenmaterial mit jedem neuen Implantat tiefer reichen muss. Eine ausreichende Stabilität des Gelenks kann nur gewährleistet werden, wenn noch unversehrtes, das heißt stabiles Knochenmaterial erreicht werden kann. Diesbezüglich ist zu beachten, dass die Erneuerung des Implantats nur bis zu einer durch die Substanz des Knochenmaterials begrenzten Anzahl möglich ist. Zudem kommt es bei der Revision selbst häufig zu Komplikationen und Beschädigungen der Knochensubstanz.

Insbesondere kommt es zwischen dem Implantat und der Schraube aufgrund des beim Einsetzen notwendigen Drehmoments, der spezifischen Materialeigenschaften und der zeitlich langen Verbindung zwischen Implantat und Schraube zu einem Kaltverschweißen. Bei diesem Kaltverschweißen kommt es auf Ebene der Gitterstruktur der aufeinander liegenden Bauteile zu einer Ausbildung eines gemeinsamen Atomgitters und damit einer relativ stabilen Verbindung der Bauteile. Tritt ein Kaltverschweißen zwischen Schraube und Implantat auf, ist das zur Entfernung der Schraube notwendige Drehmoment deutlich größer als jenes, das zum Einbringen der Schraube notwendig war.

Ähnliche Kräfte treten auch im Rahmen einer Platten-Osteosynthese, beispielsweise zur Stabilisierung nach Knochenbrüchen oder bei Versteifungsoperationen von Gelenken auf. Bei der Platten-Osteosynthese werden Platten mittels Schrauben am Knochen befestigt und fixieren so zwei oder mehrere Knochen oder Knochenfragmente miteinander, beispielsweise bei Knochenbrüchen, Versteifungsoperationen der Wirbelsäule, oder dergleichen. Die Verbindung zwischen der Knochenschraube und der Knochenplatte ist dabei häufig winkelstabil. Die Knochenplatten verbleiben je nach Anwendungsfall über einen längeren Zeitraum im Körper, so dass es auch hier häufig zu einem Kaltverschweißen zwischen der Knochenschraube und der Knochenplatte kommt.

Aufgrund des bei der Revision notwendigen großen Drehmoments kommt es dabei häufig zu einer Abnutzung des verwendeten Werkzeugs und der im Implantat oder Knochenplatte eingesetzten Schraube, genauer zu einem Verrunden der Außenkontur des Werkzeugs und einem Verrunden der Innenkontur der Schraube, das heißt dem Schraubenkopfantrieb. Anstatt die Verbindung zwischen Schraube und Implantat oder Knochenplatte zu lösen, wird die Kontur des Schraubenkopfantriebs vielmehr zerstört und ein Lösen aufgrund der mit dem Verrunden der Kontur einhergehenden sinkenden Kraftübertragung mit jedem Versuch immer schwieriger. Nicht selten erfolgt die Revision schließlich mittels eines Sägens, Fräsens oder Aufbohrens der Schraube. Dies führt zu einer zusätzlichen Zerstörung von Knochensubstanz und vermindert so beispielsweise die Stabilität nachfolgender Implantate.

Die Revision von Implantaten oder Knochenplatten erfolgt häufig mittels eines herkömmlichen Standartwerkzeugs. Diese Werkzeuge lassen sich jedoch nicht - oder nur unter erheblichem Aufwand - mit den in der Orthopädie notwendigen Anforderungen in Einklang bringen. Insbesondere müssen etwa die bei dem Einsetzen des Implantats oder der Knochenplatte notwendigen Kräfte fein justiert werden können und die verwendeten Werkzeuge derart entsprechend steril gehalten werden können, dass der Patient keinen Bakterien ausgesetzt ist.

### Stand der Technik

Um die aufzubringende Kraft, genauer das Drehmoment, besser kontrollieren zu können offenbart beispielsweise die EP 0 222 971 A1 einen Schraubendreher für chirurgische Zwecke. Der Schraubendreher weist hierbei eine zweiteilige Ausgestaltung des Handgriffs auf, wodurch der rückwärtige Griffteil sicher in der Hand gehalten werden kann und der vordere, drehbare Griffteil, mit Daumen und Zeigefinger feinfühlig gedreht werden kann. Durch die bessere Kontrolle des Drehmoments soll hier ein Beschädigen der Schraube durch ein Überdrehen und ein Ausreißen der Schraube aus dem Knochen vermieden werden. Ein weiterer Schraubenzieher für chirurgische Zwecke ist Gegenstand der US 2011/0098715 A1.

Zur sicheren Übertragung eines großen Drehmoments offenbart weiter die DE 20 2013 105 409 U1 eine Knochenschraube sowie ein entsprechend ausgebildetes Werkzeug. In dem Schraubenkopf ist eine Werkzeugaufnahme vorgesehen, welche in einem Scheitel einen Klemmbereich aufweist, der beidseitig von einem Schraubbereich flankiert ist. Dem Klemmbereich der Knochenschraube entsprechend sind ebenso am Werkzeug Klemmbereiche ausgebildet, so dass die Knochenschraube sowie das Werkzeug aufeinander abgestimmte Klemmbereiche aufweisen. Bei dem in dieser Druckschrift vorgeschlagenem Lösungsansatz muss die Knochenschraube immer in Verbindung mit dem entsprechenden, speziell für diese Knochenschraube vorgesehenen Werkzeug verwendet werden. Eine Knochenschraube und ein hierzu passendes Werkzeug ist zudem Gegenstand der US 2011/0270323 A1.

Ein Stoßinstrument für ein chirurgisches Implantat mit den Merkmalen des Oberbegriffs des Anspruchs 1 ist schließlich aus der EP 2 732 776 A1 bekannt.

Im Stand der Technik ist somit häufig nur eine bestimmte Kombination von Werkzeug und Schraube möglich. Diese Revisionssets können daher nicht universell, das heißt bei verschiedenartigen künstlichen Gelenken verwendet werden. Nachteilhaft ist dabei insbesondere die Beschränkung auf die vorgesehenen Schraubengrößen, was häufig zu Kompromissen und damit nicht zu der Verwendung der für den jeweiligen Patienten passendsten Schraubengröße führt. Zudem kann häufig nicht davon ausgegangen werden, dass derselbe Arzt das Implantat setzt und dieses wieder entfernt. Muss das Implantat etwa ungeplant, beispielsweise bei einem Unfall oder einer Entzündung, schnell entfernt werden, ist es nicht praktikabel, zuerst die notwendigen Daten über die verwendeten Schrauben, beziehungsweise Revisionssets, in Erfahrung bringen zu müssen.

Darüber hinaus wird das Problem der gegenseitigen Abnutzung zwischen Schraube und Werkzeug im Stand der Technik nicht thematisiert. Die auftretende Problematik, dass im Rahmen der Revision größere Drehmomente aufgrund eines Kaltverschweißens zwischen Schraube und Implantat notwendig sind, bleibt somit außer Acht. Somit kann es auch bei der Verwendung spezieller Revisionssets bei der Revision zu einem gegenseitigen Abnutzen kommen, was schließlich ein Verrunden des Schraubenkopfantriebs hervorruft und somit eine schwer zu lösende Verbindung verursacht. Dies führt zu einer für den Arzt komplizierten sowie zeitintensiven und für den Patienten nachteiligen Revisionsprozedur mittels Aufbohren, Sägen oder ähnlichem.

Ausgehend vom Stand der Technik liegt der Erfindung daher die Aufgabe zugrunde, eine flexibel einsetzbare Vorrichtung auszubilden, mit welcher eine bei einem Implantat oder einer Knochenplatte verbaute Schraube sicher und einfach gelöst werden kann.

### Kurzfassung der Erfindung

Diese Aufgabe wird durch eine Vorrichtung zum Lösen von Schrauben in der Orthopädie mit den Merkmalen des Anspruchs 1 gelöst.

Die erfindungsgemäße Vorrichtung zum Lösen von, insbesondere kaltverschweißten, Schrauben in der Orthopädie, weist ein Gehäuse, das in Axialrichtung des Gehäuses betrachtet eine längliche, vorzugsweise röhrenartige Kontur aufweist, und zumindest ein Schlagelement, einen Stempel, eine erste Feder und eine Werkzeugaufnahme darin beweglich gelagert aufnimmt, auf, wobei die Werkzeugaufnahme an einem in Axialrichtung des Gehäuses betrachtet vorderen Ende des Gehäuses in dem Gehäuse aufgenommen ist und über ein Lager an dem Gehäuse gehalten ist, an einem in Axialrichtung des Gehäuses betrachtet, dem vorderen Ende gegenüberliegenden hinteren Ende des Gehäuses ein Verschlusselement ausgebildet ist, das Schlagelement und der Stempel miteinander in Verbindung stehen, zur Ausbildung einer Schlaggruppe, die erste Feder zwischen einer in Axialrichtung des Gehäuses betrachtet hinteren Endfläche der Schlaggruppe und einer in Richtung zur Schlaggruppe weisenden inneren Endfläche des Verschlusselements angeordnet ist, zumindest eine Außenführungsschiene in zumindest einem Teilbereich eines Außenumfangs des Gehäuses axial verlaufend ausgebildet ist, entlang welcher zumindest ein Vorspannelement entlang der Axialrichtung des Gehäuses beweglich geführt ist, zumindest eine Innenführungsschiene in zumindest einem Teilbereich eines Innenumfangs des Gehäuses axial verlaufend ausgebildet ist, in der zumindest ein einstückig an der Schlaggruppe ausgebildetes Eingriffelement geführt ist, das mit dem zumindest einem Vorspannelement in Eingriff bringbar ist, die an der hinteren Endfläche der Schlaggruppe anliegende erste Feder durch Verschieben des an dem Eingriffelement angreifenden Vorspannelements in Richtung zur Endfläche vorspannbar ist, wodurch die in dem Gehäuse beweglich geführte Schlaggruppe in Richtung zur Endfläche verschoben wird, und die erste Feder bei Erreichen eines Scheitelpunktes zwischen dem Eingriffelement und dem Vorspannelement schlagartig auslösbar ist, zur Übertragung eines Impulses auf die Werkzeugaufnahme vermittels der Schlaggruppe derart, dass die Werkzeugaufnahme ansprechend auf den Impuls über das Lager in Umfangsrichtung verdrehbar ist, so dass die dabei erzeugte, impulsartige Drehbewegung vermittels eines in der Werkzeugaufnahme aufgenommenen Werkzeugeinsatzes auf eine zu lösende Schraube übertragbar ist, die mit dem Werkzeugeinsatz in Verbindung steht.

Durch die erfindunsgemäße Vorrichtung kann auf eine beispielsweise bei einem Implantat oder einer Knochenplatte verwendete Schraube ein großes Drehmoment aufgebracht werden. Durch den in der Vorrichtung ausgebildeten Mechanismus erfolgt die Kraftübertragung dabei schlagartig. Mit anderen Worten steigt das von der erfinderischen Vorrichtung auf die Schraube übertragene Drehmoment sprunghaft an. Die in der Vorrichtung angeordnete erste Feder wird durch einen Stempel komprimiert. Durch das Komprimieren, das heißt dem Spannen der ersten Feder, wird deren potentielle Energie erhöht. Die Feder liegt dabei an dem beweglichen Stempel an, beziehungsweise wirkt auf diesen. Wird dieser Stempel nun mittels eines an diesem eingreifenden Vorspannelements in seiner Lage gegen die Federkraft verschoben, erhöht die bei dem Verschieben des Stempels zurückgelegte Strecke somit die potentielle Energie der ersten Feder. Die Erhöhung der potentiellen Energie findet solange statt, bis das Vorspannelement einen Scheitelpunkt erreicht. Nach dem Überschreiten des Scheitelpunktes greift das Vorspannelement nicht länger über ein an dem Stempel ausgebildetes Eingriffelement ein. Mit anderen Worten wird der Stempel nach dem Überschreiten des Scheitelpunktes nicht mehr von dem Vorspannelement geführt und gehalten. Die auf den Stempel wirkende und durch das Verschieben desselben vorgespannte Feder kann sich nun entspannen. Diese Entspannung findet dabei schlagartig statt, da der Stempel nach Überschreiten des Scheitelpunktes in dem Gehäuse beschleunigt werden kann. Das Lösen des Stempels aus dem Eingriff des Vorspannelements erfolgt unmittelbar nach dem Überschreiten des Scheitelpunktes.

Die potentielle Energie der Feder wird nach dem Überschreiten des Scheitelpunktes auf den Stempel in Form von kinetischer Energie übertragen. Der Stempel wird somit durch die Federkraft der ersten Feder beschleunigt. Mit anderen Worten wird der Stempel durch die Umwandlung der potentiellen Energie der ersten Feder zu kinetischer Energie des Stempels in Bewegung versetzt, wodurch er unter Berücksichtigung seiner Masse einen Impuls besitzt. Dieser Impuls des Stempels wird durch das Auftreffen des Stempels auf das Schlagelement und weiter, durch die Kontaktfläche zwischen Schlagelement und Werkzeugaufnahme, auf diese übertragen. Durch die entsprechend ausgestaltete Kontaktfläche wird die Werkzeugaufnahme schlagartig, das heißt durch einen sprunghaften Anstieg des übertragenen Impulses, verdreht.

Durch dieses schlagartige Verdrehen, mit anderen Worten der plötzlichen Beschleunigung der Werkzeugaufnahme in Umfangsrichtung, wird ein hoher Drehimpuls erzeugt. Durch diesen hohen Drehimpuls wird auf die Schraube kurzzeitig ein großes Drehmoment übertragen. Dieses Drehmoment ist dabei so groß, dass es die zwischen der Schraube und dem Gegengewinde des Implantats oder der Knochenplatte entstandene Verbindung zerstören kann. Im Rahmen des Kaltverschweißens ist zwischen der Oberflächen der Schraube und des Gegengewindes unter Ausbildung eines gemeinsamen Atomgitters eine stabile Verbindung entstanden. Um diese Verbindung zu überwinden, reicht ein Verschieben der Oberflächen um wenige Millimeter aus, so dass sich die Atome gegeneinander verschieben. Dieser Effekt wird durch das auf die Schraube wirkende kurzzeitig große Drehmoment erreicht. Hierdurch wird ein sicheres Lösen der Schraube gewährleistet ohne dabei zu einem Verrunden des Schraubenkopfantriebs zu führen.

Wie aufgezeigt wurde, kann durch ein Kaltverschweißen, das zwischen Implantat und Schraube auf Ebene der Gitterstruktur aufgrund beispielsweise einer zeitlich langen Verbindung auftritt, eine starke Verbindung entstehen. Aufgrund dieser starken Verbindung ist die anfänglich notwendige Kraft zum Lösen dieser Verbindung höher als das beim Eindrehen oder Anziehen der Schraube aufgebrachte Drehmoment. Durch die erfindungsgemäße Vorrichtung ist es möglich, auf die Schraube einen Drehimpuls, mit anderen Worten ein schlagartig ansteigendes Drehmoment aufzubringen. Durch diesen Impuls kann die Verbindung, das heißt eine Verbindung der Oberflächen, zerstört werden. Das nachfolgende Ausdrehen der Schraube erfordert kein erhöhtes Drehmoment mehr und kann so mühelos vollzogen werden. Die Schraube kann so sicher gelöst werden, ohne dass es für den Patienten zu negativen Folgen kommt.

Insbesondere erhöht das kurzzeitig aufgebrachte hohe Drehmoment die Wahrscheinlichkeit, dass die Verbindung zwischen Schraube und Implantat oder Knochenplatte bei dem ersten Versuch gelöst wird. Damit findet kein Abrutschen des Werkzeugs in dem Schraubenkopf und somit kein Verrunden des Schraubenkopfantriebs statt. Insbesondere kann so ein komplizierter Revisionsablauf vermieden werden, bei welchem die Schraube mittels Sägen oder Aufbohren entfernt werden muss. Die Knochensubstanz des Patienten kann geschont und die Operationszeit wesentlich verringert werden.

Nach einer Weiterbildung der erfindungsgemäßen Vorrichtung kann in dem Gehäuse eine zweite Feder angeordnet sein, die eine Kraft auf die Werkzeugaufnahme aufbringt und derart konfiguriert ist, dass sie die Werkzeugaufnahme nach der Drehbewegung, welche durch die Übertragung des Impulses der Schlaggruppe auf die Werkzeugaufnahme verursacht wird, in eine Ruheposition zurückstellt.

Durch das Ausbilden einer zweiten Feder als einer Positionsfeder kann die Lage der Werkzeugaufnahme sicher eingestellt werden. Somit kann sichergestellt werden, dass die Werkzeugaufnahme vor der Impulsübertragung ausreichend Spiel für eine Drehbewegung zur Verfügung hat. Es wird somit beispielsweise vermieden, dass die Vorrichtung auslöst, das heißt ein Impuls auf die Werkzeugaufnahme aufgebracht wird, ohne dass sich diese Verdrehen kann und der Impuls somit nicht in eine Drehbewegung umgewandelt wird, welche zu einem Lösen der Schraube führt. Zudem muss durch das Rückstellen der Werkzeugaufnahme durch die zweite Feder kein manuelles Rückstellen der Werkzeugaufnahme erfolgen. Das Rückstellen findet durch die zweite Feder vielmehr automatisch nach einem Auslösen der Vorrichtung statt, indem sich die zweite Feder durch die Rückstellbewegung der Werkzeugaufnahme entspannt.

Nach einer Weiterbildung der Vorrichtung können der Stempel und das Schlagelement einstückig als Schlaggruppe ausgebildet sein, und der durch die erste Feder auf die Schlaggruppe aufgebrachte Impuls kann die Schlaggruppe in axialer Richtung beschleunigen zur Übertragung eines Impulses in Umfangsrichtung auf die Werkzeugaufnahme.

Durch diese Weiterbildung kann die Anzahl der in der Vorrichtung enthaltenen Komponenten verringert werden. Indem der Stempel und das Schlagelement einstückig ausgebildet sind, übernimmt die hierdurch entstehende Schlaggruppe sowohl die Funktion des Stempels als auch des Schlagteils. Zudem kann durch diese Ausgestaltung der auf die Werkzeugaufnahme wirkende Impuls erhöht werden. Der Impuls ergibt sich aus der Formel p = kg·m·s⁻¹. Werden der Stempel und das Schlagteil als ein Element ausgebildet, kann damit die beschleunigte Masse erhöht und folglich der Impuls vergrößert werden. Das auf die Schraube schlagartig aufgebrachte Drehmoment wird durch diese Ausgestaltung erhöht und schließlich die Wahrscheinlichkeit vergrößert, dass die Verbindung zwischen Schraube und Implantat oder Knochenplatte sicher gelöst werden kann. Durch diese Ausgestaltung lässt sich somit nicht nur die Komplexität der Vorrichtung verringern sondern gleichzeitig die Effektivität der Vorrichtung erhöhen.

Nach einer Weiterbildung der Vorrichtung kann der Stempel der Schlaggruppe als an der Innenkontur des Gehäuses anliegendes Ringelement ausgebildet sein, an dessen zum Verschlusselement weisenden Ende die erste Feder angreift, und an dessen zur Werkzeugaufnahme weisenden Ende das Schlagelement in Form von zumindest einem stabförmigen Fortsatz ausgebildet ist, wobei an dem stabförmigen Fortsatz ein rampenförmiger Vorsprung ausgebildet ist, und die Werkzeugaufnahme eine Vertiefung in Umfangsrichtung aufweist, welche komplementär zu dem Vorsprung ausgebildet ist, wobei der an dem Fortsatz ausgebildete Vorsprung in einem entspannten Zustand der ersten Feder in der Vertiefung aufgenommen ist, und die in der Werkzeugaufnahme ausgebildete Vertiefung eine zur Axialrichtung des Gehäuses geneigte Fläche ausgebildete Vertiefung aufweist, entlang welcher während des Spannens der ersten Feder eine hierzu komplementär ausgebildete zur Axialrichtung des Gehäuses geneigte Fläche des an dem Fortsatz ausgebildeten Vorsprungs abgleitet.

Nach dieser Weiterbildung der Vorrichtung kann durch eine einfache konstruktive Maßnahme sichergestellt werden, dass vor einem erneuten Auslösen der Vorrichtung, genauer vor einem erneuten Auslösen der ersten Feder, ein Rückstellen der Werkzeugaufnahme in eine Ausgangsposition erfolgt. Dies geschieht durch ein entsprechendes Ausbilden zweier zumindest in einem Teilbereich einander gegenüberliegender komplementär verlaufender Flächen an der Werkzeugaufnahme und der Schlaggruppe. Vor einem erneuten Auslösen der ersten Feder muss diese in eine gespannte Position rückgestellt werden. Dies geschieht durch ein Verschieben der Schlaggruppe in Richtung zur zweiten Feder. Zeitgleich mit dieser Vorspannbewegung der Schlaggruppe erfolgt hier nun ein Rückdrehen der Werkzeugaufnahme, da die Schlaggruppe diese während der Rückstellbewegung durch ein Abgleiten der zueinander komplementär ausgebildeten Flächen automatisch verdreht. Die Rückstellbewegung kann dabei nicht erfolgen, ohne dass nicht auch die Werkzeugaufnahme verdreht wird. Durch diese einfache konstruktive Maßnahme kann so ein Fehlauslösen, das heißt ein Auslösen, ohne, dass die Werkzeugaufnahme zurückgedreht wurde, vermieden werden.

Nach einer Weiterbildung der Vorrichtung kann an dem stabförmigen Fortsatz zumindest eine zur Axialrichtung geneigte Fläche ausgebildet sein, welche bei einem Entspannen der ersten Feder derart mit der Werkzeugaufnahme in Kontakt kommt, dass die Werkzeugaufnahme durch die axiale Bewegung der Schlaggruppe in Umfangsrichtung beschleunigt wird.

Die Schlaggruppe und die Werkzeugaufnahme weisen nach dieser Weiterbildung zwei zueinander zeigende, komplementär verlaufende schräge Flächen auf. In dem Zustand der Vorrichtung, in welchem die erste Feder durch das Verschieben des Stempels komprimiert ist, das heißt die erste Feder vorgespannt ist, ist zwischen der Schlaggruppe und der Werkzeugaufnahme ein sich in axialer Richtung erstreckender Abstand ausgebildet. Wird nun die erste Feder entspannt, wird die Schlaggruppe in axialer Richtung beschleunigt und trifft anschließend auf die Werkzeugaufnahme. Durch die komplementär schräg zueinander ausgebildeten Flächen wird nun die in Axialrichtung wirkende Kraft zumindest teilweise in eine in Umfangsrichtung wirkende Kraft umgewandelt. Durch ein Auftreffen der geneigten Fläche der Schlaggruppe auf die geneigte Fläche der Werkzeugaufnahme, welche nur einen Freiheitsgrad in Umfangsrichtung besitzt, wird diese in eine Drehbewegung gezwungen. Durch diese Ausgestaltung wird eine sichere Umwandlung der in Längsrichtung der Vorrichtung wirkenden Kraft in eine Drehbewegung der Werkzeugaufnahme ermöglicht.

Nach einer Weiterbildung der Vorrichtung können der Stempel und das Schlagelement separat voneinander ausgebildet sein, und der durch die erste Feder auf den Stempel der Schlaggruppe aufgebrachte Impuls das Schlagelement der Schlaggruppe in axialer Richtung beschleunigen, zur Übertragung eines Impulses in Umfangsrichtung auf die Werkzeugaufnahme.

Nach dieser Weiterbildung ist die Schlaggruppe nach ihren funktionalen Aspekten in zwei Elemente getrennt. So fungiert ein Teil der Schlaggruppe, der Stempel, als Schwungmasse, welche durch die erste Feder beschleunigt wird. Das andere Teil der Schlaggruppe, das Schlagelement, fungiert als Verdrehelement, das die Werkzeugaufnahme in Umfangsrichtung beschleunigt. Bei dieser Weiterbildung können die Bestandteile der Schlaggruppe durch das konstruktive Trennen der Elemente, jeweils nach ihrer Hauptfunktion optimiert werden. Mit anderen Worten kann der Stempel derart optimiert werden, dass er einen möglichst hohen Impuls übertragen kann. Beispielsweise kann die Masse des Stempels erhöht werden, um einen möglichst hohen Impuls durch die durch die erste Feder verursachte Beschleunigung zu erreichen. Zudem kann die Fläche des Schlagelements auf den Verdrehmechanismus hin optimiert werden.

Nach einer Weiterbildung der Vorrichtung kann eine in Axialrichtung zur Werkzeugaufnahme weisende Übertragungsfläche des Schlagelements unter Ausbildung einer Führungsfläche stufenartig ausgebildet sein, wobei die Übertragungsfläche zu einer ihr gegenüberliegenden Aufnahmefläche der Werkzeugaufnahme komplementär ausgebildet ist, so dass bei einem Entspannen der ersten Feder die Werkzeugaufnahme durch die axiale Bewegung des Schlagelements über ein Abgleiten der Führungsfläche an einem Teil der Aufnahmefläche in Umfangsrichtung beschleunigbar ist.

Nach dieser Weiterbildung weist das Schlagelement eine zur Axialrichtung geneigte Endfläche auf, welche der Werkzeugaufnahme gegenüberliegt. Zudem weist ebenso die Werkzeugaufnahme eine zur Axialrichtung geneigte Endfläche auf, welche dem Schlagelement gegenüberliegt. In einem gespannten, das heißt komprimierten Zustand der ersten Feder sind diese beiden Flächen in Umfangsrichtung zueinander versetzt angeordnet. Trifft das Schlagelement auf die Werkzeugaufnahme auf, zwingt das Schlagelement durch ein Abgleiten der Endflächen der Werkzeugaufnahme, das heißt der Aufnahmefläche, auf der Endfläche des Schlagelements, das heißt der Führungsfläche, die Werkzeugaufnahme in eine Rotation. Durch diese einfache konstruktive Ausgestaltung, das heißt die robuste Konstruktion, ist der Mechanismus wenig fehleranfällig und kann so sicher gewährleistet werden.

Nach einer Weiterbildung der Vorrichtung kann zumindest ein Hebelelement über zumindest ein Gelenk an dem mit dem Gehäuse verbundenen Verschlusselement angebracht sein, und das zumindest eine Hebelelement kann über das zumindest eine Gelenk relativ zu dem Gehäuse bewegbar sein, wobei das zumindest eine Hebelelement derart konfiguriert ist, dass es bei einer Bewegung in Richtung zum Gehäuse das zumindest eine Eingriffelement axial in Richtung zur hinteren Endfläche verschiebt.

Durch die Ausbildung eines Hebelelements als Mechanismus zum manuellen Spannen der ersten Feder kann die zum Vorspannen gegen die Federkraft notwendige Kraft gering gehalten werden. Unter Ausnutzung eines großen Hebelarms lässt sich bei dieser Ausgestaltung die notwendige Kraft um ein Vielfaches geringer einstellen, als die gegen die Feder aufzubringende Kraft. Zudem kann durch diese Ausbildung die zum Vorspannen notwendige Kraft benutzerfreundlich auf die Vorrichtung aufgebracht werden. Der Benutzer muss zum Vorspannen der ersten Feder nur eine Greifbewegung durchführen. Genauer kann der Benutzter den zumindest einen Hebelarm betätigen indem er die Vorrichtung in seiner Hand zusammendrückt. Die Vorrichtung ist durch diese Ausgestaltung einfach mit einer Hand zu betätigen.

Nach einem weiteren, jedoch nicht beanspruchten Aspekt wird ein Werkzeugeinsatz zum Lösen von, insbesondere kaltverschweißten, Schrauben in der Orthopädie, insbesondere zur Verwendung mit einer Vorrichtung nach den oben beschriebenen Aspekten ausgebildet. Der Werkzeugeinsatz weist einen Werkzeugkorpus mit einem zumindest teilweise polygonal ausgebildeten Abschnitt, mittels welchem der Werkzeugeinsatz in der Werkzeugaufnahme gehalten werden kann, und eine Werkzeugspitze auf, die mit einer Schraube in Eingriff bringbar ist, um diese durch Beaufschlagen mit einer von dem Schlagelement auf die Werkzeugaufnahme der Vorrichtung übertragenen Impulskraft zu lösen, wobei die Werkzeugspitze an ihrer Innenseite eine sich zumindest teilweise in Richtung zum Werkzeugkorpus erstreckende, umlaufende, muldenartige Vertiefung aufweist, zur Aufnahme von die zu lösende Schraube umgebenden Restgewebes.

Durch die Ausgestaltung des Werkzeugeinsatzes kann dieser schnell in einer entsprechenden Vorrichtung aufgenommen und somit ebenso auch schnell gewechselt werden. Der zur Aufnahme in einer Vorrichtung ausgebildete Abschnitt ist zur radialen Kraftübertragung polygonal ausgebildet. Axial wird der Werkzeugeinsatz reibkraftschlüssig in der Vorrichtung gehalten, so dass bei einem Wechsel der sich in der Vorrichtung befindende Werkzeugeinsatz herausgezogen und der neue Werkzeugeinsatz eingesteckt werden kann. Dies ermöglicht einen schnellen Wechsel verschiedener Werkzeugeinsätze. Durch die erfindungsgemäße muldenartige Vertiefung der Werkzeugspitze kann zudem sichergestellt werden, dass die Werkzeugspitze in ausreichendem Maß auf die Schraube gesteckt werden kann. Sich noch über dem Schraubenkopf befindendes Gewebe kann in der muldenartigen Vertiefung aufgenommen werden und steht so einem Aufstecken des Werkzeuges über die Schraube nicht im Weg.

### Kurze Beschreibung der Zeichnung

Die Merkmale und Vorteile sowie die technische und wirtschaftliche Bedeutung beispielhafter Ausführungsformen der vorliegenden Erfindung werden nachfolgend Bezug nehmend auf die beigefügte Zeichnung beschrieben, hierbei zeigt:
- Fig. 1: eine perspektivische Ansicht einer ersten Ausführungsform einer erfindungsgemäßen Vorrichtung zum Lösen von Schrauben in der Orthopädie in einem ersten Zustand;
- Fig. 2: eine Querschnittsansicht der ersten Ausführungsform der erfindungsgemäßen Vorrichtung in dem ersten Zustand;
- Fig. 3: eine perspektivische Ansicht der ersten Ausführungsform der erfindungsgemäßen Vorrichtung in einem zweiten Zustand;
- Fig. 4: eine perspektivische Ansicht der Vorrichtung zum Lösen von Schrauben;
- Fig. 5: eine Querschnittsansicht einer zweiten Ausführungsform der erfindungsgemäßen Vorrichtung;
- Fig. 6: eine Prinzipzeichnung der zweiten Ausführungsform der erfindungsgemäßen Vorrichtung;
- Fig. 7: eine perspektivische Ansicht der zweiten Ausführungsform der erfindungsgemäßen Vorrichtung;
- Fig. 8: eine Teilansicht eines vorderen Bereichs der erfindungsgemäßen Vorrichtung;
- Fig. 9: eine Querschnittsansicht eines Werkzeugeinsatzes;
- Fig. 10: eine perspektivische Ansicht des Werkzeugeinsatzes;

### Detaillierte Beschreibung der Ausführungsform

Nachfolgend werden Ausführungsformen der Erfindung im Detail mit Bezug auf die beigefügten Zeichnungen beschrieben werden.

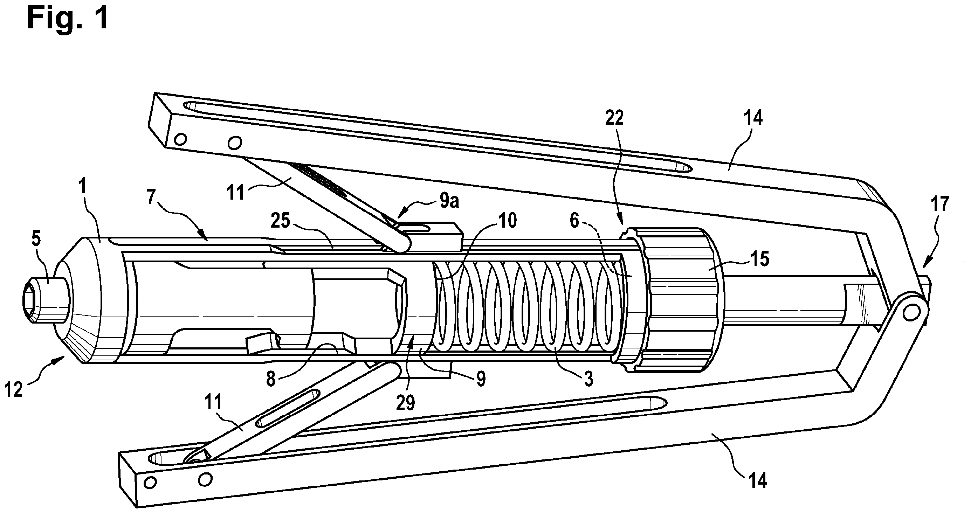

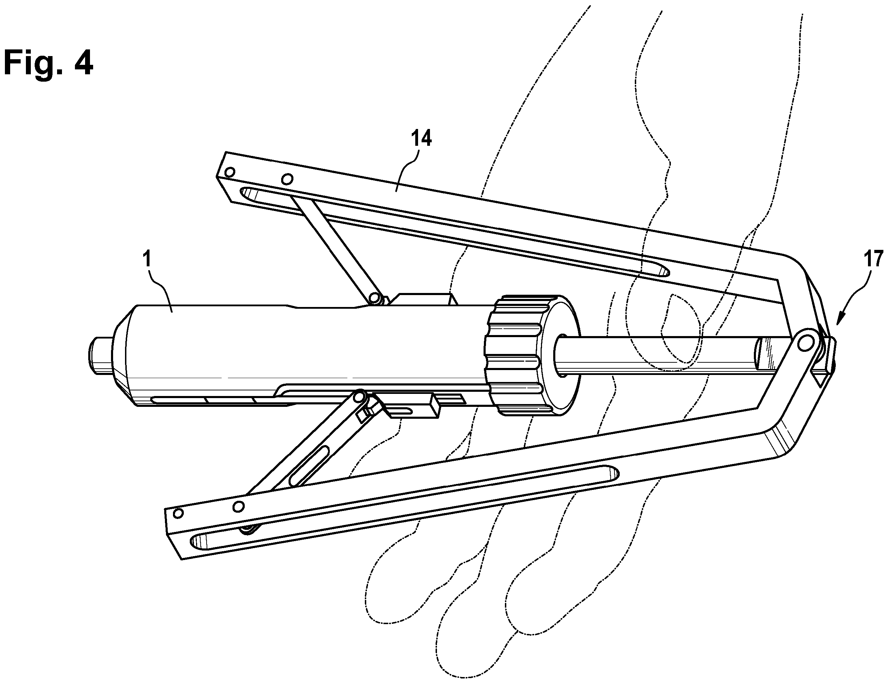

Dabei zeigt Fig. 1 eine Ausführungsform einer Vorrichtung zum Lösen von Schrauben in der Orthopädie. Die Vorrichtung weist ein Gehäuse 1 auf, an dem an einer in Axialrichtung liegenden hinteren Endseite über ein Gelenk 17, beispielsweise ein Drehgelenk, zwei Hebelelemente 14 - ähnlich den Hebelarmen eines Korkenziehers - angeordnet sind. Diese Hebelelemente 14 erstrecken sich schenkelartig zu beiden Seiten des Gehäuses 1 und können mittels des Gelenks 17 zu dem Gehäuse 1 hin und von diesem weg bewegt werden. Weiter sind an diesen Hebelelementen 14 an der von dem Gelenk 17 entfernten Seite Vorspannelemente 11 angebracht. Diese Vorspannelemente 11 sind ebenfalls gelenkig, beispielsweise auch über Drehgelenke, mit den Hebelelementen 14 verbunden. In Verwendung der Vorrichtung kann diese in der Hand gehalten werden, wie beispielsweise in Fig. 4 dargestellt ist, und über ein Bewegen der Hebelelemente 14 mit einer Hand, das heißt einem Zusammendrücken der Hebelelemente 14, betätigt werden.

Durch die Bewegung der Hebelelemente 14 werden die Vorspannelemente 11 in an zwei gegenüberliegenden Seiten in Axialrichtung an dem Gehäuse 1 verlaufenden Außenführungsschienen 7 geführt. Genauer werden die Vorspannelemente 11 bei einer Bewegung der Hebelelemente 14 in Richtung zum Gehäuse 1 nach hinten, das heißt in Richtung zum Gelenk 17, geführt. Die Vorspannelemente 11 greifen dabei in Eingriffelemente 9a ein. Diese Eingriffelemente 9a sind an einem Stempel 9 einstückig, beispielsweise verschweißt oder verschraubt, ausgebildet. Der Stempel 9 ist innerhalb des Gehäuses 1 in Axialrichtung verschiebbar gelagert und kann über das Verschieben der Vorspannelemente 11 in den Außenführungsschienen 7 in Axialrichtung in dem Gehäuse 1 bewegt werden. Dabei ist an einer hinteren Seite des Stempels 9, das heißt einer Seite in Richtung zum Gelenk 17, eine erste Feder 3 in dem Gehäuse 1 angeordnet. Mit anderen Worden ist die erste Feder 3 zwischen einer nach hinten zeigenden Stirnfläche, das heißt der hinteren Endfläche 10, des Stempels 9 und einer inneren Endfläche 6 eines Verschlusselements 15 angeordnet. Die erste Feder 3 ist dabei derart in dem Gehäuse 1 angeordnet, dass sie durch ein Verschieben des Stempels 9 ausgelenkt werden kann.

Wie in der in Fig. 1 gezeigten Ausführungsform dargestellt ist, kann die erste Feder 3 als Druckfeder ausgebildet sein. Je nach Konstruktion der Vorrichtung kann die erste Feder 3 aber auch als Tellerfeder, Biegefeder, Spiralfeder oder Luftfeder ausgebildet sein. Die erste Feder 3 wird in der hier gezeigten Ausführungsform in zumindest einem Teilbereich ihres Außenumfangs durch einen sich in Axialrichtung erstreckenden Bereich des Gehäuses 1 geführt, so dass die erste Feder 3 bei einer Kompression nicht seitlich, das heißt quer zur Krafteinwirkrichtung ausweichen kann. Die Federkonstante der ersten Feder 3 ist dabei derart eingestellt, dass sie entsprechend der zur Verfügung stehenden Wegänderung bei einer Auslenkung ausreichend potentielle Energie speichern kann, so dass sie bei einer Entspannung den Stempel 9, beziehungsweise die Schlaggruppe 29, in Axialrichtung beschleunigen kann.

Die erste Feder 3 wird durch eine Verschiebung des Stempels 9 ausgelenkt. Die bei der Verschiebung zurückgelegte Wegstrecke des Stempels 9 entspricht in dieser Ausführungsform der Längenänderung der ersten Feder 3. Dabei kann die erste Feder 3 aber auch eine geringere Längenänderung im Vergleich zu der Wegstrecke des Stempels 9 erfahren. Beispielsweise muss die erste Feder 3 nicht direkt an der hinteren Endfläche 6 und Stirnfläche 10 des Stempels 9 anliegen. Zudem kann die erste Feder 3 eine lineare Kennlinie, aber auch eine progressive oder degressive Kennlinie, aufweisen. Ebenso kann die erste Feder 3 aus mehreren Federn bestehen, die in Reihe oder parallel geschalten sind.

In der hier dargestellten Ausführungsform ist die erste Feder 3 in das Gehäuse 1 lose eingelegt. Dabei kann die erste Feder 3 jedoch auch an ihren Enden mit der hinteren Endfläche 6 des Gehäuses 1 sowie der Endfläche 10 des Stempels 9 fest verbunden, beispielsweise verklebt oder verschweißt, sein. Der Stempel 9 ist in dem Gehäuse 1 über Innenführungsschienen 8 axial verschiebbar ausgebildet. Hierfür sind an dem Stempel 9 Eingriffelemente 9a ausgebildet, welche sich radial von dem Stempel 9 nach außen erstrecken. Diese Eingriffelemente 9a verlaufen in der Innenführungsschiene 8 nach außen, so dass sie von dem Außenumfang des Gehäuses 1 hervorstehen, um ein Eingreifen der Vorspannelemente 11 zu erlauben. Wie in Fig. 1 gezeigt ist, sind hier zwei sich gegenüberliegende Innenführungsschienen 8 ausgebildet. Alternativ können aber auch mehrere Innenführungsschienen 8 sowie nur eine Innenführungsschiene 8 ausgebildet sein. In dem Gehäuse 1 ist weiter ein Schlagelement 2 angeordnet. Auch das Schlagelement 2 ist axial verschiebbar in dem Gehäuse 1 angeordnet.

Dabei kann das Schlagelement 2 ebenso wie der Stempel 9 in den Innenführungsschienen 8 geführt werden. Es können aber auch für das Schlagelement 2 weitere Führungsschienen an dem Innenumfang des Gehäuses 1 ausgebildet sein. In der in Fig. 1 gezeigten Ausführungsform sind das Schlagelement 2 und der Stempel 9 einstückig, als Schlaggruppe 29, ausgebildet. Diese Elemente, das heißt das Schlagelement 2 und der Stempel 9, können aber auch wie später beschrieben, zweistückig ausgebildet sein. Der Schlaggruppe 29 axial nachfolgend, das heißt weg von der ersten Feder 3, ist eine Werkzeugaufnahme 5 angeordnet. Die Werkzeugaufnahme 5 ist in dem Gehäuse 1 um die Axialrichtung rotierbar, nicht aber axial verschiebbar ausgebildet.

In dem Gehäuse 1 sind somit ausgehend von einem hinteren Ende, an welchem das Verschlusselement 15 angebracht ist, der Reihe nach zumindest die erste Feder 3, die Schlaggruppe 29 sowie die Werkzeugaufnahme 5 ausgebildet. Die Werkzeugaufnahme 5 steht dabei in axialer Richtung aus dem Gehäuse 1 hervor. Mit anderen Worten ist an einer vorderen Endseite 12 des Gehäuses 1 eine Durchgangsöffnung ausgebildet, durch welche ein Teil der Werkzeugaufnahme 5, genauer der Teil in welcher ein Werkzeug aufgenommen werden kann, aus dem Gehäuse 1 ragt. In der Fig. 1 ist ein erster Zustand dargestellt, in welcher die erste Feder 3 teilweise durch den Stempel 9 der Schlaggruppe 29 ausgelenkt ist.

Genauer ist hier ein Zustand gezeigt, in welchem die Hebelelemente 14 bereits teilweise in Richtung zum Gehäuse 1 gedrückt wurden, so dass der Stempel 9 durch die Vorspannelemente 11 geführt, nicht mehr mit der Werkzeugaufnahme 5 in Kontakt ist, sondern in Richtung zur ersten Feder 3 verschoben wurde. Je weiter in dieser Ausführungsform die Hebelelemente 14 in Richtung zum Gehäuse 1 gedrückt werden, das heißt je kleiner der zwischen Hebelelementen 14 und Gehäuse 1 ausgebildete Abstand wird, desto weiter wird die erste Feder 3 ausgelenkt und so ihre potentielle Energie erhöht. An den Hebelelementen 14 sind hierfür Vorspannelemente 11 gelenkig ausgebildet, welche bei einer Bewegung der Hebelelemente 14 durch diese weiter an den Außenführungsschienen 7 in Richtung zur ersten Feder 3 geschoben, beziehungsweise gedrückt werden.

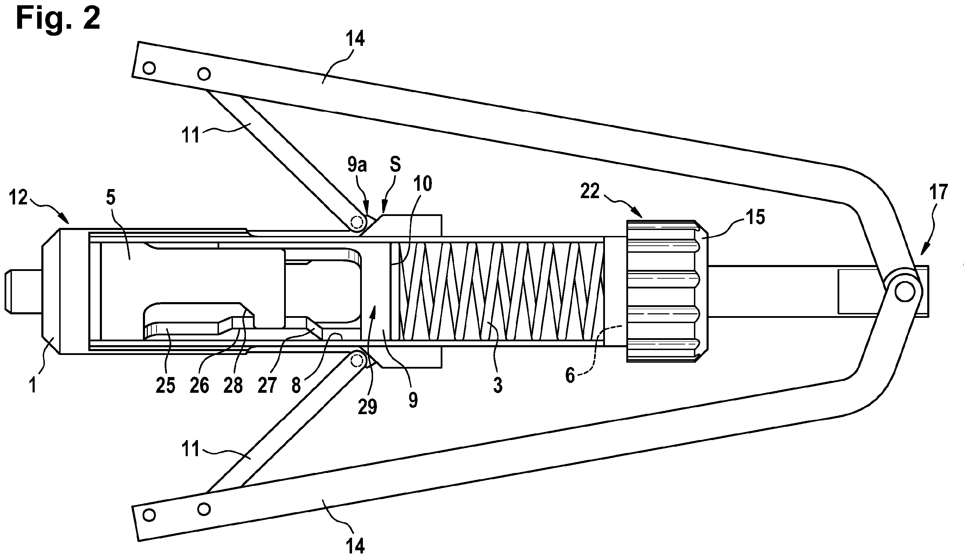

Fig. 2 zeigt eine Querschnittsansicht der ersten Ausführungsform der erfindungsgemäßen Vorrichtung in dem ersten Zustand. Wie oben beschrieben ist, ist die erste Feder 3 in dem ersten Zustand teilweise durch ein Verschieben der Schlaggruppe 29 in Richtung zum Verschlusselement 15 vorgespannt. Mit anderen Worten ist zwischen der Schlaggruppe 29 und der Werkzeugaufnahme 5 ein Abstand in Axialrichtung ausgebildet, in welchem die Schlaggruppe 29 in Richtung zur Werkzeugaufnahme 5 beschleunigt werden kann. Überschreitet das Vorspannelement 11 den Scheitelpunkt S, welcher hier als rampenförmiger Vorsprung am Außenumfang des Gehäuses 1 ausgebildet ist, so wird das Eingriffelement 9a nicht länger durch das Vorspannelement 11 gehalten.

Mit anderen Worten wird das Vorspannelement 11 in dieser Ausführungsform durch den rampenförmigen Vorsprung soweit in Radialrichtung nach außen von dem Gehäuse 1 weg ausgelenkt, dass das Vorspannelement 11 nicht mehr in das Eingriffelement 9a des Stempels 9 der Schlaggruppe 29 eingreift. Da nach dem Überschreiten des Scheitelpunktes S keine Kraft entgegen der Federkraft der ersten Feder 3 mehr an dem Eingriffelement 9a und damit dem Stempel 9 angreift, wird die Schlaggruppe 29 durch Entspannen der ersten Feder 3 in Richtung zur Werkzeugaufnahme 5 beschleunigt. An der Schlaggruppe 29 ist eine zur Axialrichtung geneigte Fläche ausgebildet, welche auf eine entsprechend komplementär ausgebildet geneigte Fläche der Werkzeugaufnahme 5 auftrifft. Da die aufeinander treffenden Flächen schräg zur Axialrichtung ausgebildet sind, findet hier sowohl eine Kraftübertragung in Axialrichtung sowie in Umfangsrichtung statt. Die Werkzeugaufnahme 5 kann allerdings, wie in Fig. 2 gezeigt, nur in Umfangsrichtung bewegt werden, so dass der Impuls der Schlaggruppe 29 in einen Drehimpuls der Werkzeugaufnahme 5 gewandelt wird.

Zusätzlich gelangt in dieser Ausführungsform ein Vorsprung 26, der an dem Fortsatz 25 der Schlaggruppe 29 ausgebildet ist, in eine Vertiefung der Werkzeugaufnahme 5. Die Vertiefung der Werkzeugaufnahme 5 ist in Umfangsrichtung ausgebildet. Bei einem Verschieben der Schlaggruppe 29, nachdem die Werkzeugaufnahme 5 verdreht worden ist, von der Werkzeugaufnahme 5 weg, das heißt bei einem Spannen der ersten Feder 3, dreht die Schlaggruppe 29 die Werkzeugaufnahme 5 in ihre Ausgangsposition zurück. Dies geschieht durch ein Abgleiten einer zur Axialrichtung geneigten Fläche 28 die an einem hinteren Ende der Vertiefung ausgebildet ist. Da die Schlaggruppe 29 nur axial verschiebbar ist und die Werkzeugaufnahme 5 nur in Umfangsrichtung verdrehbar ist, wird durch das Abgleiten der geneigten Fläche 28 der Werkzeugaufnahme 5 auf einer komplementär geneigten Fläche 27 der Schlaggruppe 29 die Werkzeugaufnahme 5 rotiert. Mit anderen Worten bewirkt die Schlaggruppe 29 bei einer Bewegung in Richtung zur Werkzeugaufnahme 5 ein Rotieren der Werkzeugaufnahme 5 in eine erste Richtung und bewirkt bei einer Bewegung von der Werkzeugaufnahme 5 weg ein Rotieren der Werkzeugaufnahme 5 in die zweite, entgegengesetzte Richtung.

Wie in Fig. 2 gezeigt ist, ist der stabförmige Fortsatz 25 durch zwei um 180° Grad versetzt zueinander ausgebildete, sich in Axialrichtung des Gehäuses 1 erstreckende Elemente ausgebildet. Die Anzahl der sich erstreckenden Elemente kann dabei aber auch nur eines oder mehrere sein. Zudem kann der Vorsprung 26 nur an einem Teil der Anzahl von sich erstreckenden Elementen ausgebildet sein, so dass nicht alle sich erstreckenden Elemente der Schlaggruppe 29 einen Vorsprung 26 aufweisen.

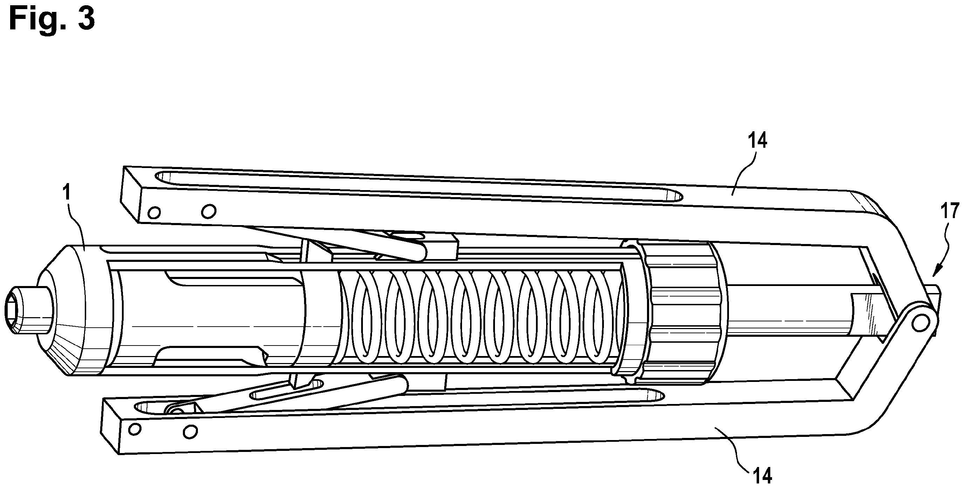

Fig. 3 zeigt einen zweiten Zustand der ersten Ausführungsform der Vorrichtung. In diesem zweiten Zustand wird die erste Feder 3 nicht mehr durch das Verschieben der Schlaggruppe 29 mittels der Vorspannelemente 11 vorgespannt. Vielmehr haben die Vorspannelemente 11 den Scheitelpunkt S überschritten, so dass sie nach dem Überwinden des Scheitelpunktes S ohne die Schlaggruppe 29 über die Eingriffelemente 9a zu verschieben, axial entlang des Gehäuses 1 geführt werden, beispielsweise solange bis die Hebelelemente 14 nicht weiter in Richtung zum Gehäuse 1 geführt werden können, da sie beispielsweise an dem Verschlusselement 15 anstoßen. In der hier gezeigten Ausführungsform drückt die erste Feder 3 die Schlaggruppe 29 auch in den zweiten Zustand noch gegen die Werkzeugaufnahme 5, daher muss die erste Feder 3 in dem zweiten Zustand nicht vollständig entspannt sein.

Insbesondere kann die erste Feder 3 durch das Anliegen an der Endfläche 6 und der Endfläche 10 noch in einem gespannten Zustand sein. Alternativ muss die erste Feder 3 aber nicht an den beiden Endflächen 6 oder 10 anliegen, sondern kann lose, ähnlich der Feder in einem Kugelschreiber, angeordnet sein. Zudem kann die erste Feder 3 auch mit einer Endfläche 6 oder 10 fest verbunden sein, so dass ein Abstand in Axialrichtung der ersten Feder 3 zu nur einer Endfläche 6 oder 10 ausgebildet wird. Der zweite Zustand der Vorrichtung ist daher insbesondere dadurch definiert, dass der Stempel 9 der Schlaggruppe 29 nicht mehr durch das Vorspannelement 11 gegen die erste Feder 3 gedrückt wird. Die potentielle Energie der ersten Feder 3 ist in dem ersten Zustand größer als in dem zweiten Zustand der Vorrichtung. Wie hier in Fig. 3 dargestellt ist, können die Hebelelemente 14 soweit in Richtung zum Gehäuse 1 geführt werden bis sie an das Verschlusselement 15, beispielsweise als eine auf das Gehäuse 1 aufgeschraubte Verschlusskappe 15 ausgeführt, anstoßen. Der Bewegungsfreiraum der Hebelelemente 14 kann aber auch durch andere Elemente, beispielsweise durch eine entsprechende Ausgestaltung des Gelenks 17, beschränkt werden.

Wie oben beschrieben ist, zeigt Fig. 4 die Vorrichtung, wenn sie in der Hand gehalten wird. Das Material der gesamten Vorrichtung 1 ist derart ausgewählt, dass es möglichst steril gehalten werden kann. Dabei ist die Form der Vorrichtung, das heißt insbesondere des Gehäuses 1 durch eine glatte Oberfläche und weitgehend frei von Hinterschneidungen, Spalten und ähnlichem geprägt. Zudem sind die Schnittstellen der gesamten Vorrichtung gering gehalten, so dass das Gehäuse 1 vorzugsweise durchgehend geschlossen verläuft und nur für den Austritt der Werkzeugaufnahme 5 eine Öffnung sowie eine Schnittstelle zum Aufsetzen des Verschlusselements 15 aufweist. Das Material der gesamten Vorrichtung ist weiter aus chirurgischem Material wie Edelstahl, Titan oder Tantal gewählt und kann leicht, beispielsweise mittels Autoklavierung, sterilisiert werden. Zudem kann die erste und/oder zweite Feder aus einem nicht rostenden Federstahldraht nach EN 10270-3 oder unlegiertem Federstahldraht gefertigt sein. Um die Ergonomie der Vorrichtung zu erhöhen, kann diese durch Verwenden von Leichtmetall wie eloxiertem Aluminium ergänzt werden.

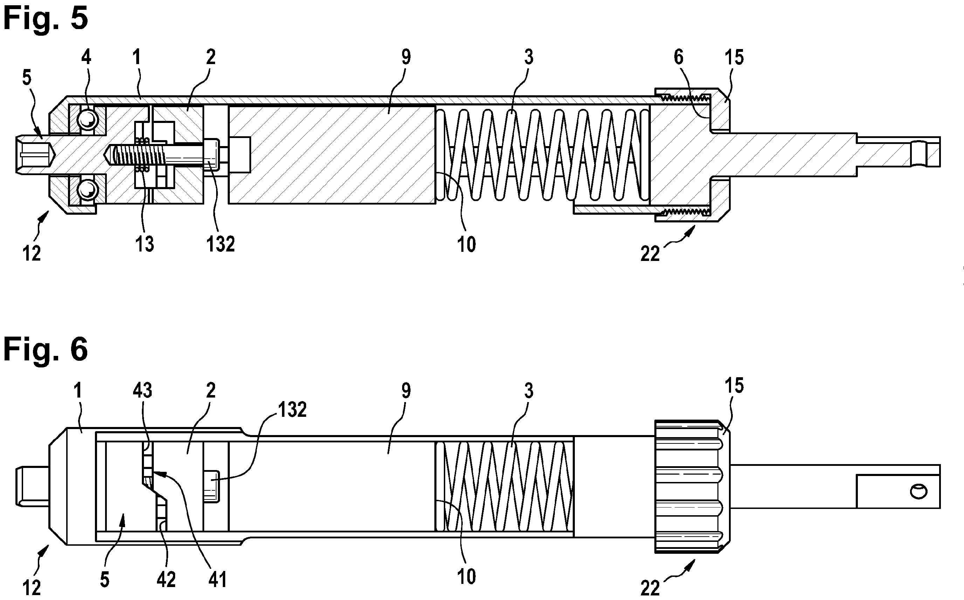

In Fig. 5 ist eine Querschnittsansicht einer zweiten Ausführungsform der erfindungsgemäßen Vorrichtung gezeigt. In dieser Ausführungsform sind der Stempel 9 und das Schlagelement 2 als voneinander getrennte Elemente ausgebildet. Zusätzlich ist in der Vorrichtung ein Kugellager 4 ausgebildet, welches die Werkzeugaufnahme 5 gegen das Gehäuse 1 lagert, um so eine Rotation der Werkzeugaufnahme 5 bei möglichst geringer Reibung zu ermöglichen. Das Lager 4 kann wie hier dargestellt als Kugellager, aber ebenso als Gleitlager, Kegellager oder ähnliches, ausgebildet sein. Weiter ist wie in Fig. 5 zu sehen, eine zweite Feder 13 in dem Gehäuse 1 angeordnet. Diese zweite Feder 13 wird über einen Positionsstift 132 annähernd auf der Achse des Gehäuses 1, um welche ebenso das Lager 4 angeordnet ist und sich die Werkzeugaufnahme 5 dreht, gehalten.

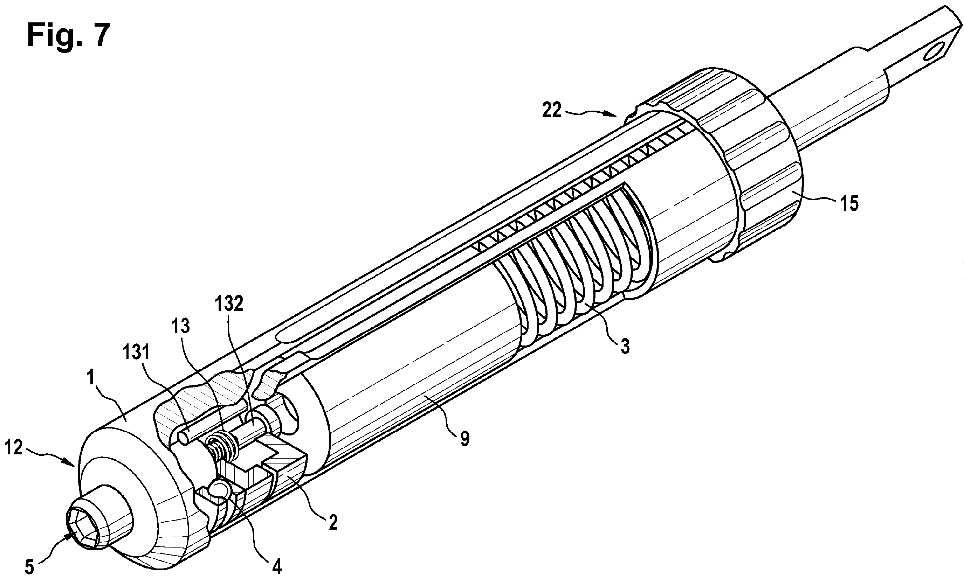

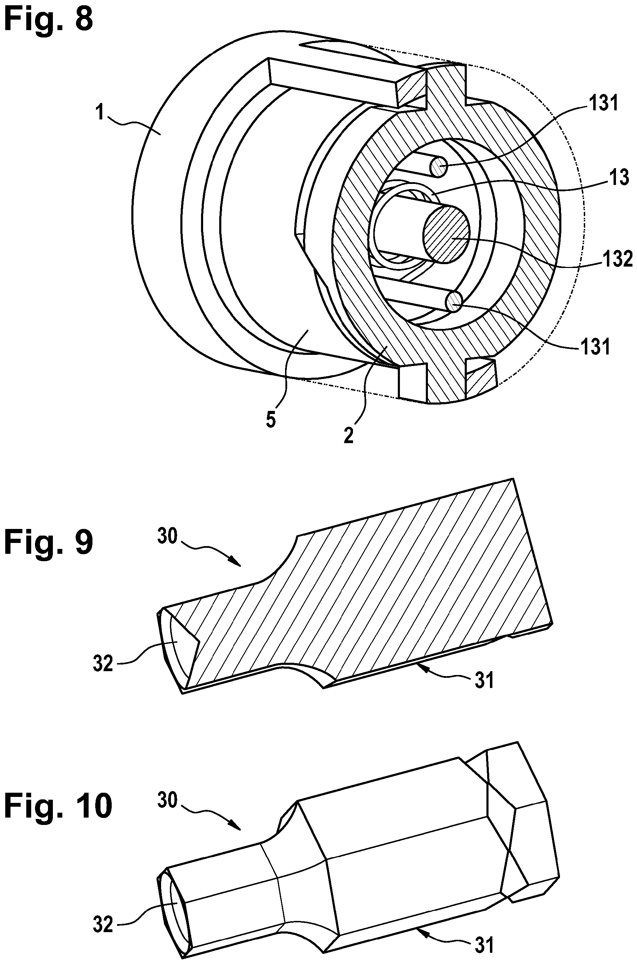

Der Positionsstift 132 kann wie hier dargestellt ist, durch das Schlagelement 2 verlaufen und in der Werkzeugaufnahme 5 verschraubt sein. Alternativ kann der Positionsstift 132 auch auf oder in die Werkzeugaufnahme 5 pressgepasst oder verklebt werden. Die zweite Feder 13 ist als Torsionsfeder ausgebildet, welche die Werkzeugaufnahme 5 nach ihrer Rotation durch das Schlagelement 2 hervorgerufen, wieder in ihre Ausgangsposition zurückstellt. Diese Ausgangsposition der Werkzeugaufnahme 5 wird, wie in Fig. 7 und Fig. 8 gezeigt ist, durch das Ausbilden von weiteren Positionsstiften 131 erreicht, welche die Verdrehung der zweiten Feder 13 beschränken.

Fig. 6 zeigt die Ausgestaltung der Schlaggruppe 29 der zweiten Ausführungsform als zwei voneinander getrennte Elemente. Die erste Feder 3 wird hier durch nur ein Verschieben des Stempels 9 über entsprechend der ersten Ausführungsform ausgebildete Eingriffelemente 9a, an welchen die Vorspannelemente 11 eingreifen, vorgespannt, um anschließend den Stempel 9, nachdem die Vorspannelemente 11 den Scheitelpunkt S überschritten haben, zu beschleunigen, so dass der Stempel 9 seinen Impuls auf das Schlagelement 2 übertragen kann. Dabei wird das Schlagelement 2 nur in Axialrichtung des Gehäuses 1 durch den Stempel 9 beschleunigt. Der von dem Stempel 9 auf das Schlagelement 2 übertragene Impuls wird von dem Schlagelement 2 weiter auf die Werkzeugaufnahme 5 übertragen. Dabei besitzt die Werkzeugaufnahme 5 keinen Impuls in Axialrichtung sondern nur einen Drehimpuls. Diese Umwandlung der Kraft in Axialrichtung des Schlagelements 2 in eine Kraft in Umfangsrichtung der Werkzeugaufnahme 5 wird ähnlich der ersten Ausführungsform durch zwei komplementär zueinander, schräg zur Axialrichtung verlaufende Flächen von Schlagelement 2 und Werkzeugaufnahme 5 erreicht.

Im Unterschied zu der ersten Ausführungsform erfolgt hier das Rückstellen der Werkzeugaufnahme 5 durch die zweite Feder 13. Durch das Rückstellen der Werkzeugaufnahme 5 wird so ebenfalls die Schlaggruppe 29, das heißt Schlagelement 2 und Stempel 9 wieder in Axialrichtung von der Werkzeugaufnahme 5 weg bewegt, da hier ähnlich die beiden schräg zur Axialrichtung verlaufenden Flächen aneinander abgleiten. Dabei geht bei der Rückstellung der Werkzeugaufnahme 5 und weiter des Schlagelements 2 in die Ausgangsposition die Kraftübertragung nicht von der ersten Feder 3 sondern von der zweiten Feder 13 aus. Mit anderen Worten zwingt bei einer Entspannung der ersten Feder 3 das Schlagelement 2 die Werkzeugaufnahme 5 in eine Rotation während bei einer Entspannung der zweiten Feder 13 die Werkzeugaufnahme 5 das Schlagelement 2 in eine Axialbewegung zwingt.

Obwohl die zweite Feder 13 und das Lager 4 oben nur im Rahmen der zweiten Ausführungsform beschrieben sind, können diese Elemente ebenso bei der ersten Ausführungsform verwendet werden. Dabei kann die durch den rampenförmigen Vorsprung 26 veranlasste Rückstellbewegung der Werkzeugaufnahme 5 durch die zweite Feder 13 verstärkt werden. Zudem kann bei der ersten Ausführungsform das Lager 4 verwendet werden, ohne dass hier eine zweite Feder 13 ausgebildet ist. Ähnlich muss bei der zweiten Ausführungsform kein Lager ausgebildet sein.

In Fig. 9 und Fig. 10 ist schließlich ein Werkzeugeinsatz 30 in Fig. 9 im Querschnitt sowie in Fig. 10 perspektivisch dargestellt. Wie in Fig. 10 gezeigt ist, ist der Außenumfang des Werkzeugeinsatzes 30 polygonal, vorzugsweise sechseckig, ausgebildet, so dass er in die Werkzeugaufnahme 5, welche einen komplementär ausgebildeten polygonalen Innenumfang aufweist, eingesteckt werden kann. Durch die polygonale Form ist eine formschlüssige Kraftübertragung zwischen Werkzeugeinsatz 30 und Werkzeugaufnahme 5 möglich. Dabei ist der Außenumfang des Werkzeugeinsatzes 30 in einem hinteren Bereich, welcher in der Werkzeugaufnahme 5 der Vorrichtung aufgenommen wird, größer ausgebildet, als der Außenumfang der Werkzeugspitze 32. Hierdurch kann eine große Fläche zur Kraftübertragung von der Vorrichtung über den Werkzeugeinsatz 30 auf die im Implantat oder einer Knochenplatte eingesetzte Schraube gewährleistet werden. Der Werkzeugeinsatz 30 ist vorzugsweise aus einem Vollmaterial, beispielsweise chirurgischem Material wie Edelstahl, Titan oder Tantal, geformt.

Weiter ist eine Werkzeugspitze 32, die in eine entsprechende Schraube eingesteckt werden kann, derart ausgebildet, dass eine muldenartige Vertiefung in der Werkzeugspitze 32 verläuft. Durch diese Vertiefung kann verhindert werden, dass sich noch über der Schraube befindendes Gewebe eine Kraftübertragung zwischen Schraube und Werkzeugspitze 32 stört, indem das Gewebe in dieser Vertiefung bei dem Einstecken der Werkzeugspitze 32 in die Schraube nach hinten verdrängt wird. Der Werkzeugeinsatz 30 muss hier nur in einem Teilbereich an den Werkzeugeinsatz 30 angepasst sein, so dass eine große Anzahl möglicher Variationen der Werkzeugspitze 32 zugelassen werden. Je nach Schraube kann daher ein anderer Werkzeugeinsatz 30 verwendet, und einfach in die Werkzeugaufnahme 5 der Vorrichtung eingesteckt werden.

Es wird eine Vorrichtung zum Lösen von, insbesondere kaltverschweißten, Schrauben in der Orthopädie vorgeschlagen. Dabei weist die Vorrichtung ein Gehäuse 1 mit einem darin beweglich gehaltenen Schlagelement 2, einem beweglichen Stempel 9 und einer ersten Feder 3 auf. Weiter sind an dem Gehäuse 1 zumindest eine Außenführungsschiene 7, entlang der zumindest ein Vorspannelement 11 beweglich gelagert ist, und eine Innenführungsschiene 8, in der zumindest ein an dem Stempel 9 ausgebildetes Eingriffelement 9a geführt wird, ausgebildet. Die erste Feder 3 kann dabei durch Verschieben des an dem Eingriffelement 9a des Stempels 9 angreifenden Vorspannelements 11 gegen die hintere Endfläche 6 vorgespannt werden. Weiter ist an einem vorderen Ende 12 des Gehäuses 1 eine Werkzeugaufnahme 5 ausgebildet, die über ein Lager 4 an dem Gehäuse 1 gehalten ist. Ferner wird ein Werkzeugeinsatz 30 zur Verwendung mit der Vorrichtung vorgeschlagen.

## Patentansprüche

1. Vorrichtung zum Lösen von, insbesondere kaltverschweißten, Schrauben in der Orthopädie, aufweisend:
ein Gehäuse (1), das in Axialrichtung des Gehäuses (1) betrachtet eine längliche, vorzugsweise röhrenartige Kontur aufweist, und zumindest ein Schlagelement (2), einen Stempel (9), eine erste Feder (3) und eine Werkzeugaufnahme (5) darin beweglich gelagert aufnimmt, wobei
die Werkzeugaufnahme (5) an einem in Axialrichtung des Gehäuses (1) betrachtet vorderen Ende (12) des Gehäuses (1) in dem Gehäuse (1) aufgenommen ist und über ein Lager (4) an dem Gehäuse (1) gehalten ist,
an einem in Axialrichtung des Gehäuses (1) betrachtet, dem vorderen Ende (12) gegenüberliegenden hinteren Ende (22) des Gehäuses (1) ein Verschlusselement (15) ausgebildet ist,
das Schlagelement (2) und der Stempel (9) miteinander in Verbindung stehen, zur Ausbildung einer Schlaggruppe (29),
die erste Feder (3) zwischen einer in Axialrichtung des Gehäuses (1) betrachtet hinteren Endfläche (10) der Schlaggruppe (29) und einer in Richtung zur Schlaggruppe (29) weisenden hinteren Endfläche (6) des Verschlusselements (15) angeordnet ist,
**dadurch gekennzeichnet, dass**
zumindest eine Außenführungsschiene (7) in zumindest einem Teilbereich eines Außenumfangs des Gehäuses (1) axial verlaufend ausgebildet ist, entlang welcher zumindest ein Vorspannelement (11) entlang der Axialrichtung des Gehäuses (1) beweglich geführt ist,
zumindest eine Innenführungsschiene (8) in zumindest einem Teilbereich eines Innenumfangs des Gehäuses (1) axial verlaufend ausgebildet ist, in der zumindest ein einstückig an der Schlaggruppe (29) ausgebildetes Eingriffselement (9a) geführt ist, das mit dem zumindest einem Vorspannelement (11) in Eingriff bringbar ist,
die an der hinteren Endfläche (10) der Schlaggruppe (29) anliegende erste Feder (3) durch Verschieben des an dem Eingriffelement (9a) angreifenden Vorspannelements (11) in Richtung zur Endfläche (6) vorspannbar ist, wodurch die in dem Gehäuse (1) beweglich geführte Schlaggruppe (29) in Richtung zur Endfläche (6) verschoben wird, und
die erste Feder (3) bei Erreichen eines Scheitelpunktes (S) zwischen dem Eingriffelement (9a) und dem Vorspannelement (11) schlagartig auslösbar ist, zur Übertragung eines Impulses auf die Werkzeugaufnahme (5) vermittels der Schlaggruppe (29) derart, dass die Werkzeugaufnahme (5) ansprechend auf den Impuls über das Lager (4) in Umfangsrichtung verdrehbar ist, so dass die dabei erzeugte, impulsartige Drehbewegung vermittels eines in der Werkzeugaufnahme (5) aufgenommenen Werkzeugeinsatzes (30) auf eine zu lösende Schraube übertragbar ist, die mit dem Werkzeugeinsatz in Verbindung steht.

2. Vorrichtung nach Anspruch 1, wobei
in dem Gehäuse (1) eine zweite Feder (13) angeordnet ist, die eine Kraft auf die Werkzeugaufnahme (5) aufbringt, und derart konfiguriert ist, dass sie die Werkzeugaufnahme (5) nach der Drehbewegung, welche durch die Übertragung des Impulses der Schlaggruppe (29) auf die Werkzeugaufnahme (5) verursacht wird, in eine Ruheposition zurückstellt.

3. Vorrichtung nach Anspruch 1 oder 2, wobei
der Stempel (9) und das Schlagelement (2) einstückig als Schlaggruppe (29) ausgebildet sind, und
der durch die erste Feder (3) auf die Schlaggruppe (29) aufgebrachte Impuls die Schlaggruppe (29) in axialer Richtung beschleunigt, zur Übertragung eines Impulses in Umfangsrichtung auf die Werkzeugaufnahme (5).

4. Vorrichtung nach einem der Ansprüche 1 bis 3, wobei
der Stempel (9) der Schlaggruppe (29) als an der Innenkontur des Gehäuses (1) anliegendes Ringelement ausgebildet ist, an dessen zum Verschlusselement (15) weisenden Ende die erste Feder (3) angreift, und an dessen zur Werkzeugaufnahme (5) weisenden Ende das Schlagelement (2) in Form von zumindest einem stabförmigen Fortsatz (25) ausgebildet ist, wobei an dem stabförmigen Fortsatz (25) ein rampenförmiger Vorsprung (26) ausgebildet ist, und die Werkzeugaufnahme (5) eine Vertiefung in Umfangsrichtung aufweist, welche komplementär zu dem Vorsprung (26) ausgebildet ist,
wobei der an dem Fortsatz (25) ausgebildete Vorsprung (26) in einem entspannten Zustand der ersten Feder (3) in der Vertiefung aufgenommen ist, und
die in der Werkzeugaufnahme (5) ausgebildete Vertiefung eine zur Axialrichtung des Gehäuses (1) geneigte Fläche (28) ausgebildete Vertiefung aufweist, entlang welcher während des Spannens der ersten Feder (3) eine hierzu komplementär ausgebildete zur Axialrichtung des Gehäuses (1) geneigte Fläche (27) des an dem Fortsatz (25) ausgebildeten Vorsprungs (26) abgleitet.

5. Vorrichtung nach Anspruch 4, wobei an dem stabförmigen Fortsatz (25) zumindest eine zur Axialrichtung geneigte Fläche ausgebildet ist, welche bei einem Entspannen der ersten Feder (3) derart mit der Werkzeugaufnahme (5) in Kontakt kommt, dass die Werkzeugaufnahme (5) durch die axiale Bewegung der Schlaggruppe (29) in Umfangsrichtung beschleunigt wird.

6. Vorrichtung nach Anspruch 1 oder 2, wobei
der Stempel (9) und das Schlagelement (2) separat voneinander ausgebildet sind, und
der durch die erste Feder (3) auf den Stempel (9) der Schlaggruppe (29) aufgebrachte Impuls das Schlagelement (2) der Schlaggruppe (29) in axialer Richtung beschleunigt, zur Übertragung eines Impulses in Umfangsrichtung auf die Werkzeugaufnahme (5).

7. Vorrichtung nach Anspruch 6, wobei
eine in Axialrichtung zur Werkzeugaufnahme (5) weisende Übertragungsfläche (41) des Schlagelements (2) unter Ausbildung einer Führungsfläche (42) stufenartig ausgebildet ist, wobei die Übertragungsfläche (41) zu einer ihr gegenüberliegenden Aufnahmefläche (43) der Werkzeugaufnahme (5) komplementär ausgebildet ist, so dass bei einem Entspannen der ersten Feder (3) die Werkzeugaufnahme (5) durch die axiale Bewegung des Schlagelements (2) über ein Abgleiten der Führungsfläche (42) an einem Teil der Aufnahmefläche (43) in Umfangsrichtung beschleunigbar ist.

8. Vorrichtung nach einem der Ansprüche 1 bis 7, wobei
zumindest ein Hebelelement (14) über zumindest ein Gelenk (17) an dem mit dem Gehäuse (1) verbundenen Verschlusselement (15) angebracht ist, und das zumindest eine Hebelelement (14) über das zumindest eine Gelenk (17) relativ zu dem Gehäuse (1) bewegbar ist, wobei das zumindest eine Hebelelement (14) derart konfiguriert ist, dass es bei einer Bewegung in Richtung zum Gehäuse (1) das zumindest eine Eingriffelement (9a) axial in Richtung zur hinteren Endfläche (6) verschiebt.

## Claims

1. Apparatus for loosening in particular cold welded, screws in orthopaedics, having:
a housing (1) which has, as viewed in the axial direction of the housing (1), an elongate, preferably tubular contour, and receives at least one impact element (2), a plunger (9), a first spring (3) and a tool receptacle (5) movably mounted therein, wherein
the tool receptacle (5) is received in the housing (1) at a front end (12) of the housing (1), as viewed in the axial direction of the housing (1), and is held on the housing (1) via a bearing (4),
a closing element (15) is provided at a rear end (22) of the housing (1) opposite, as viewed in the axial direction of the housing (1), the front end (12),
the impact element (2) and the plunger (9) are connected to one another to form an impact group (29),
the first spring (3) is arranged, as viewed in the axial direction of the housing (1), between a rear end face (10) of the impact group (29) and a rear end face (6) of the closing element (15), facing in the direction toward the impact group (29),
**characterised in that**
at least one outer guide rail (7) is provided extending axially in at least a subregion of an outer periphery of the housing (1), along which outer guide rail at least one preloading element (11) is movably guided along the axial direction of the housing (1),
at least one inner guide rail (8) is provided extending axially in at least a subregion of an inner periphery of the housing (1), in which inner guide rail an engaging element (9a) integrally formed on the impact group (29) is guided, which engaging element is bringable into engagement with the at least one preloading element (11),
the first spring (3) abutting the rear end face (10) of the impact group (29) is preloadable by displacement in the direction toward the end face (6) of the preloading element (11) engaging on the engaging element (9a), by which means the impact group (29) movably guided in the housing (1) is displaced in the direction toward the end face (6), and
on reaching the apex point (S) between the engaging element (9a) and the preloading element (11), the first spring (3) is releasable suddenly in order to transfer a momentum to the tool receptacle (5) by means of the impact group (29) such that the tool receptacle (5) is rotatable via the bearing (4) in the circumferential direction in response to the momentum, so that the impulsive rotary movement thereby generated is transferrable by means of a tool insert (30) received in the tool receptacle (5) to a screw that is to be loosened and which is in connection with the tool insert.

2. Apparatus according to claim 1, wherein
arranged in the housing (1) is a second spring (13) which exerts a force on the tool receptacle (5) and is configured so that, after the rotation movement which is caused by the transfer of the momentum of the impact group (29) to the tool receptacle (5), it restores the tool receptacle (5) into a rest position.

3. Apparatus according to claim 1 or 2, wherein
the plunger (9) and the impact element (2) are formed as a single piece as an impact group (29), and
the momentum applied by the first spring (3) to the impact group (29) accelerates the impact group (29) in the axial direction to transfer a momentum in the circumferential direction to the tool receptacle (5).

4. Apparatus according to one of the claims 1 to 3, wherein
the plunger (9) of the impact group (29) is configured as a ring element abutting the interior contour of the housing (1) on the end of which ring element facing the closing element (15) the first spring (3) engages, and on the end of which ring element facing the tool receptacle (5) the impact element (2) is formed in the form of at least one rod-shaped extension (25), wherein a ramp-shaped projection (26) is formed on the rod-shaped extension (25) and the tool receptacle (5) has a recess in the circumferential direction which is formed complementary to the projection (26),
wherein the projection (26) formed on the extension (25) is received, in a relaxed state of the first spring (3), in the recess, and
the recess formed in the tool receptacle (5) has a surface (28) [formed recess] inclined to the axial direction of the housing (1), along which surface, during the tensioning of the first spring (3), a surface (27) of the projection (26) formed on the extension (25) to be complementary thereto and inclined to the axial direction of the housing (1) slides.

5. Apparatus according to claim 4, wherein
at least one surface inclined to the axial direction is formed on the rod-shaped extension (25), which surface, on a relaxation of the first spring (3) comes into contact with the tool receptacle (5) in such a way that the tool receptacle (5) is accelerated in the circumferential direction by the axial movement of the impact group (29).

6. Apparatus according to claim 1 or 2, wherein
the plunger (9) and the impact element (2) are formed separately from one another, and
the momentum applied by the first spring (3) to the plunger (9) of the impact group (29) accelerates the impact element (2) of the impact group (29) in the axial direction to transfer a momentum in the circumferential direction to the tool receptacle (5).

7. Apparatus according to claim 6, wherein
a transfer surface (41) of the impact element (2) facing in the axial direction toward the tool receptacle (5) is designed step-shaped, forming a guide surface (42), wherein the transfer surface (41) is formed complementary to a receiving surface (43) complementary thereto of the tool receptacle (5), so that on relaxation of the first spring (3), the tool receptacle (5) is configured to be accelerated in the circumferential direction by the axial movement of the impact element (2) by means of a sliding of the guide surface (42) on a part of the receiving surface (43).

8. Apparatus according to one of the claims 1 to 7, wherein
at least one lever element (14) is mounted via at least one joint (17) on the closing element (15) connected to the housing (1) and the at least one lever element (14) is movable relative to the housing (1) by means of the at least one joint (17), wherein the at least one lever element (14) is configured such that, on a movement in the direction toward the housing (1), it displaces the at least one engaging element (9a) axially in the direction toward the rear end face (6).

## Revendications

1. Dispositif de desserrage de vis notamment soudées à froid en orthopédie comprenant :
- un boîtier (1) qui, dans la direction axiale du boîtier (1), a un contour allongé de préférence tubulaire et qui loge de manière mobile au moins un élément de frappe (2), un tampon (9), un premier ressort (3) et une prise d'outil (5),
- la prise d'outil (5) étant reçue dans le boîtier (1), à l'extrémité avant (12) du boîtier (1) selon la direction axiale du boîtier (1) et tenue au boîtier (1) par un palier (4),
- l'extrémité arrière (22) du boîtier (1) à l'opposé de l'extrémité avant (12) selon la direction axiale du boîtier (1), comporte un élément de fermeture (15),
- l'élément de frappe (2) et le tampon (9) sont reliés pour former un module de frappe (29),
- le premier ressort (3) est prévu entre une surface d'extrémité arrière (10) du module (29) selon la direction axiale du boîtier (1) et la surface d'extrémité arrière (6) de l'élément de fermeture (15) tournée en direction du module de frappe (29),
dispositif **caractérisé en ce que**
- au moins un rail de guidage extérieur (7) est réalisé selon un tracé axial dans au moins un zone partielle de la périphérie extérieure du boîtier (1), rail le long duquel au moins un élément de précontrainte (11) est guidé de manière mobile selon la direction axiale du boîtier (1),
- au moins un rail de guidage intérieur (8) est réalisé dans au moins une zone partielle de la périphérie intérieure du boîtier (1) selon un tracé axial, rail dans lequel est guidé au moins un élément de prise (9a) réalisé en une seule pièce sur le module de frappe (29), cet élément pouvant être mis en prise avec au moins un élément de précontrainte (11),
- le premier ressort (3) appliqué contre la surface d'extrémité arrière (10) du module de frappe (29) se précontraint par coulissement de l'élément de précontrainte (11) agissant sur l'élément de prise (9a) en direction de la surface d'extrémité (6), de sorte que le module de frappe (29) guidé de manière mobile dans le boîtier (1) se déplace en direction de la surface d'extrémité (6), et
- le premier ressort (3) lorsqu'il atteint le sommet (S) entre l'élément de prise (9a) et l'élément de précontrainte (11) est déclenché de manière brusque pour transmettre une impulsion à la prise d'outil (5) par l'intermédiaire du module de frappe (29) de façon que la prise d'outil (5) tourne dans la direction périphérique en réponse à l'impulsion par le palier (4) de façon que le mouvement de rotation impulsionnel ainsi généré soit transmis par l'intermédiaire d'une garniture d'outil (30) logée dans la prise d'outil (5) sur une vis à desserrer, qui est en liaison avec la garniture d'outil.

2. Dispositif selon la revendication 1,
selon lequel
un second ressort (13) est prévu dans le boîtier (1) qui applique une force sur la prise d'outil (5) et configuré pour qu'après le mouvement de rotation qui est produit par la transmission de l'impulsion du module de frappe (29) sur la prise d'outil (5), elle rappelle la prise d'outil (5) en position repos.

3. Dispositif selon la revendication 1 ou 2,
dans lequel
le tampon (9) et l'élément de frappe (2) sont réalisés en une seule pièce comme module de frappe (29), et l'impulsion appliquée par le premier ressort (3) sur le module de frappe (29), accélère le module de frappe (29) dans la direction axiale pour transmettre une impulsion dans la direction périphérique sur la prise d'outil (5).

4. Dispositif selon l'une des revendications 1 à 3,
dans lequel
- le tampon (9) du module de frappe (29) est réalisé sous la forme d'un élément annulaire appliqué contre le contour intérieur du boîtier (1) et sur lequel agit le premier ressort (3) sur l'extrémité tournée vers l'élément de fermeture (15) et dont l'extrémité tournée vers la prise d'outil (5), l'élément de frappe (2) est réalisée sous la forme d'au moins un prolongement (25) en forme de tige,
- un relief (26) en forme de rampe est réalisé sur le prolongement (25) en forme de tige et la prise d'outil (5) comporte une cavité dans la direction périphérique, cette cavité étant de forme complémentaire à celle du relief (26),
- le relief (26) réalisé sur le prolongement (25), vient dans la cavité à l'état tendu du premier ressort (3), et
- la cavité dans la prise d'outil (5) est réalisée comme cavité ayant une surface (28) inclinée par rapport à la direction axiale du boîtier (1) le long de laquelle glisse une surface (25) de forme complémentaire, inclinée par rapport à la direction axiale du boîtier (1) et faisant partie du relief (26) réalisé sur le prolongement (25).

5. Dispositif selon la revendication (4),
dans lequel
le prolongement (25) en forme de tige a au moins une surface inclinée par rapport à la direction axiale et qui, lors de la détente du premier ressort (3), arrive en contact avec la prise d'outil (5) pour accélérer la prise d'outil (5) dans la direction périphérique par le mouvement axial du module de frappe (29).

6. Dispositif selon la revendication 1 ou 2,
dans lequel
le tampon (9) et l'élément de frappe (2) sont réalisés séparément l'un de l'autre, et
l'impulsion appliquée par le premier ressort (3) sur le tampon (9) du module de frappe (29) accélère l'élément de frappe (2) du module de frappe (29) dans la direction axiale pour transmettre une impulsion dans la direction périphérique sur la prise d'outil (5).

7. Dispositif selon la revendication 6,
dans lequel
une surface de transmission (41) de l'élément de frappe (2) tournée dans la direction axiale vers la prise d'outil (5), est de forme étagée pour réaliser une surface de guidage (42),
la surface de transmission (41) étant réalisée de façon complémentaire à une surface de réception (43) qui lui fait face sur la prise d'outil (5) lors de la détente du premier ressort (3) accélérer dans la direction axiale, la prise d'outil (5) par le mouvement axial de l'élément de frappe (2) par le glissement de la surface de guidage (42) sur une partie de la surface de réception (43).

8. Dispositif selon l'une des revendications 1 à 7,
dans lequel
au moins un élément de levier (14) est prévu par l'intermédiaire d'au moins une articulation (17) sur l'élément de fermeture (15) relié au boîtier (1), cet élément de levier (14) étant mobile par rapport au boîtier (1) par au moins une articulation (17), et cet élément de levier (14) est configuré pour qu'un mouvement en direction du boîtier (1) déplace au moins un élément de prise (9a) en direction de la surface d'extrémité arrière (6).
